(19) Europäisches Patentamt
European Patent Office
Office européen des brevets

(11) **EP 2 085 483 A1**

(12) **EUROPEAN PATENT APPLICATION**
published in accordance with Art. 153(4) EPC

(43) Date of publication:
**05.08.2009 Bulletin 2009/32**

(51) Int Cl.:
***C12Q 1/44*** *(2006.01)*     ***G01N 33/573*** *(2006.01)*

(21) Application number: **08700636.7**

(22) Date of filing: **11.01.2008**

(86) International application number:
**PCT/CN2008/000081**

(87) International publication number:
**WO 2008/067777 (12.06.2008 Gazette 2008/24)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.11.2006 CN 200610138429**

(71) Applicant: **Beijing Accb Biotech Ltd.**
**Beijing 100084 (CN)**

(72) Inventors:
• **XU, Junpu**
**Beijing 100094 (CN)**

• **CHEN, Zhao**
**Beijing 100094 (CN)**
• **ZHOU, Haimeng**
**Beijing 100094 (CN)**
• **DAI, Jie**
**Beijing 100094 (CN)**
• **HE, Biao**
**Beijing 100094 (CN)**

(74) Representative: **Dossmann, Gérard**
**Bureau D.A. Casalonga & Josse**
**Bayerstrasse 71/73**
**80335 München (DE)**

(54) **TEST KIT FOR DETECTING CANCERS**

(57) The present invention relates to a kit for assaying the activity of the human arylsulphatase or its isozymes, including 4-MUS and 4-MU. The kit can be used for initial screening, therapeutic effect monitoring and recurrence inspecting of human cancers.

**Description**

FIELD OF THE INVENTION

The present invention relates to a kit for assaying cancer or other diseases.

BACKGROUND OF THE INVENTION

Cancer is highly lethal. One factor to improve the cancer survival rate is early detection. However, current early detection assays and effective treatments for cancer have many limitations.

The incidence and development of cancer are highly correlated to irregular expression of enzymes. During cancerization of cells, the activities of enzymes and their isozymes related to cell proliferation are up-regulated. Arylsulphatase (ARS) and its isozymes that can hydrolyze arylsulphates are related to detoxification in organism. It is generally agreed that the increase of serum ARS activity is related to inflammation that is referable to distinguish tumor or non-tumor. The determination of the ARS concentration in serum can provide very useful information for clinical diagnosis, treatment and prognosis of the disease.

Some studies showed that arylsulphatase (ARS) and its isozymes are hydrolases that can catalyze the hydrolysis of arylsulphate, which have been proved to be more active in serum of patients with lung cancer than in normal human serum and their activities are reduced by treatment. The increase of activity of lysosome enzymes including ARS can be used for effective diagnosis and therapeutic effect monitoring (Wozniak, A., et al, Neoplasma,2002, 49(1): p. 10-5). Moreover, the activities of ARSA and ARSB in different histological types of human primary and secondary tumors increase significantly in almost all the primary lung cancers (Gasa, S., et al, Cancer Res,1980,40(10): p.3804-9). The ARS activities in breast cancer patients are 2-5 times of those in healthy women. The activity is reduced to normal level after surgery or chemotherapy. The activity of enzyme is correlated to the size of tumor and its metastasis (Rozwodowska, M., et al, Pol Merkuriusz Lek,2004,17(99): p.252-4). It is also found that the activity of sulphatase in breast cancer tissue is roughly double of that in normal tissue (Chetrite, G.S., et al., Anticancer Res,2005,25(4): p.2827-30). Moreover, it is found that ARS activity in stomach cancer, rectum cancer, breast cancer and skin cancer is increased by using a non-specific substrate (Dzialoszynski, L.M., et al, Clin. Chim. Acta, 1966, 14(4): p.450-3).

4-methyl umbelliferone sulfate (4-MUS) can be used as a suitable substrate for examining the activity of ARS (Uchimura, K., et al, Methods In Enzymology,2006,416: p.243). Furthermore, 4-MUS has better Km, Vmax, sensitivity and stability than other substrates, and its hydrolysate 4-methyl umbelliferone (4-MU) has higher fluorescence in alkaline solution, which is convenient for detecting. The present techniques all examine either ARS A or ARS B, but the present invention is to examine both ARS A and B enzyme activity together in one assay because 4-MUS cannot distinguish A/B (Sherman, W.R., et al, Biochem J., 1967,102: p.905).

SUMMARY OF THE INVENTION

According to the biochemical reaction of 4-MUS catalyzed by ARS enzymes, based on the principle of resultant 4-MU in reaction solution reflecting enzyme activity, we examine the fluorescence intensity of 4-MU by 365nm excitation and at wavelength of 445-450nm, and draw standard curve based on 4-MU standard product with the known concentration so as to quantificationally examine the ARS activity in serum/urine samples. The initial screening, therapeutic effect monitoring and recurrence inspecting are carried out accordingly.

The present invention relates to a kit for assaying cancer, including 4-MUS and 4-MU. Preferably, the concentration of 4-MUS in the kit is 1-10mM, the concentration of 4-MU is 100-5000nM. The buffer in the kit is sodium acetate, with preferable concentration in the range of 1-10000mM, pH value between 1 and 14, more preferable concentration in the range of 10-1000mM, pH value between 2 and 14. The stop solution in the kit is mixed solution of sodium carbonate and sodium bicarbonate or mixed solution of sodium carbonate and glycin, with preferable concentration in the range of 1-50000mM, pH value between 1 and 14, more preferable concentration in the range of 10-5000mM, pH value between 7 and 12. The precipitator in the kit is lead acetate, with preferable concentration in the range of 1%-90% (w/v), more preferable concentration in the range of 10%-40% (w/v).

The present invention can be used for screening, therapeutic effect monitoring and recurrence inspecting:

Screening in normal people; diagnosis in patients with symptoms and distinguishing benign tumors and malign tumors; determining patient's conditions and evaluating treatment plans; monitoring the curative effects of tumors; predicting recurrence and prognosis.

The tumors include: lung cancer, stomach cancer, ovary cancer, pancreas cancer, breast cancer, liver cancer, oesophagus cancer, intestines cancer, nasopharynx cancer, kidney cancer, bladder cancer, lymph cancer, cholecyst cancer, ampullae cancer, penis cancer, testicle cancer, throat cancer, hypothyroid cancer, prostate cancer, palace cancer, colorectal cancer, bile duct cancer, parenchyma sarcoma, spermatogonium tumor, Ewing's sarcoma, leukaemia, osteosarcoma, encephaloma and malignant lymphoma. More preferably are lung cancer, stomach cancer, ovary cancer, pancreas cancer, breast cancer, liver cancer, oesophagus cancer, kidney cancer, lymph cancer, colorectal cancer, leukaemia and malignant lymphoma.

The advantages of the present invention (compared with ELISA):

Because the reaction between enzymes and substrates is highly effective and consistent, the determination of enzyme activity is better than antigen-antibody reaction technique, i.e. without false positive results. At the same time, the determination of enzyme activity can better reflect the biological functions of the enzyme in tumorigenesis, i.e. the experiment results are more authentic. And the product to be examined has special excitation and detection wavelength, making the whole experiment operable.

BRIEF DESCRIPTION OF THE DRAWINGS

Fig. 1 shows the principle of the invention.
Fig. 2 shows the impact of anticoagulant agent on the activity of human serum arylsulphatase or its isozymes.
Fig. 3 shows the impact of preservatives on the activity of human serum arylsulphatase or its isozymes.
Fig. 4 is the standard curve of 4-MU and fluorescence reading.
Fig. 5 is the [4-MU]-time curve to determine reaction time with 3 serum samples.
Fig. 6 is the [4-MU]-time curve of serum samples at different diluting conditions.
Fig. 7 is the comparison between activities of arylsulphatase or its isozymes in normal human serum and serum from patients with cancer such as lung cancer.

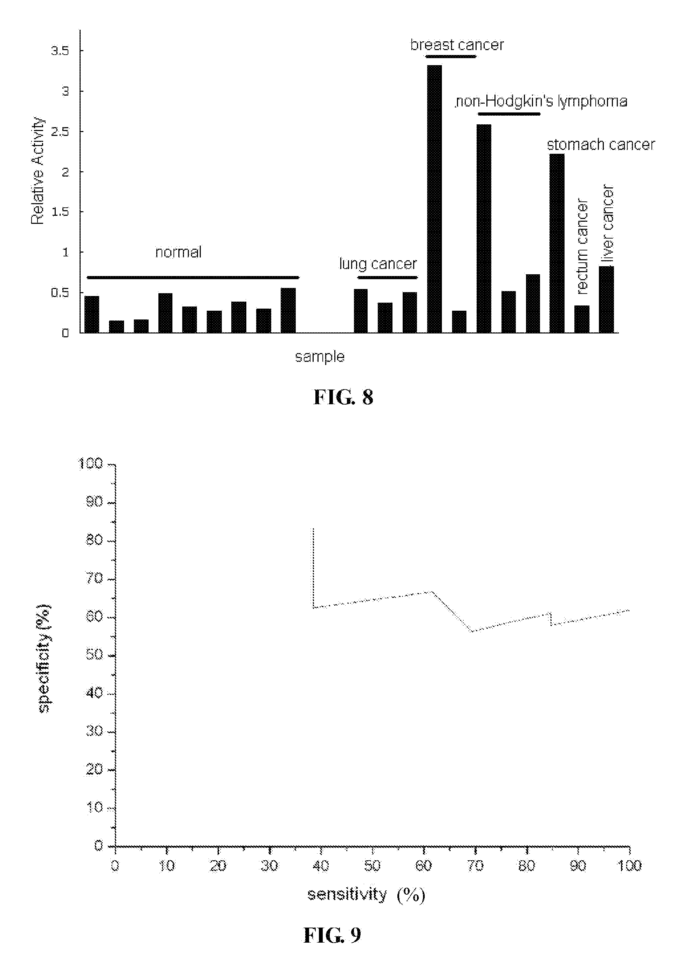

Fig. 8 is the comparison between activities of arylsulphatase or its isozymes in normal human whole blood samples and those from patients with cancer such as lung cancer.
Fig. 9 shows sensitivity-specificity corresponding to the activities of arylsulphate or its isozymes in normal human sera and sera from patients with cancer such as lung cancer.
Fig. 10 is the comparison between activities of arylsulphatase or its isozymes in normal human urine samples and those from patients with cancer.
Fig. 11 shows the sensitivity of activity increases of arylsulphatase or its isozymes in urine samples from patients with different types of cancers.
Fig. 12 is the comparison between activities of arylsulphatase or its isozymes in normal human urine samples and those from treated patients with cancer.

DETAILED DESCRIPTION OF THE INVENTION

(1) Determination of reaction parameters

1 Reaction parameters

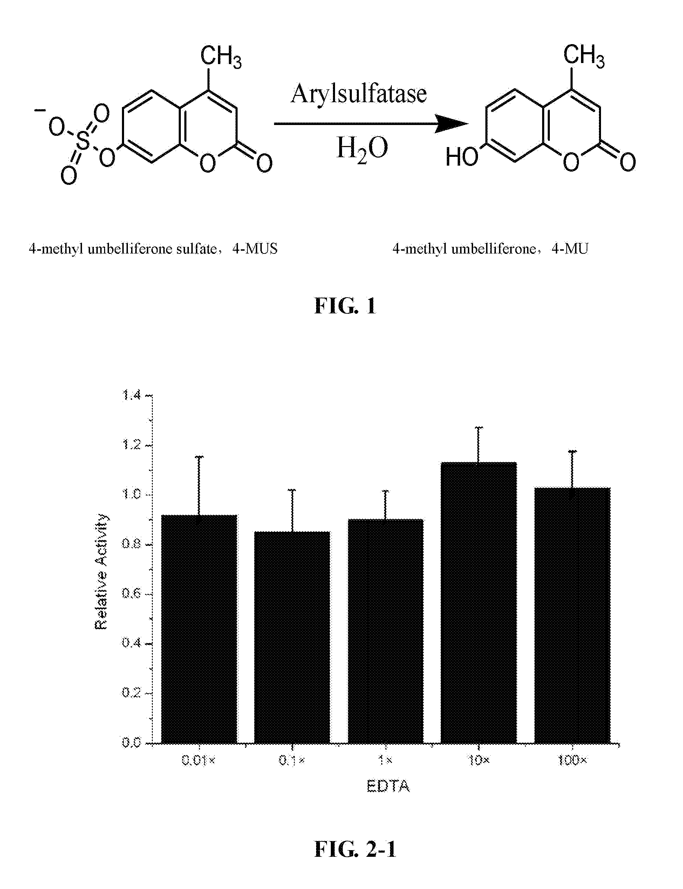

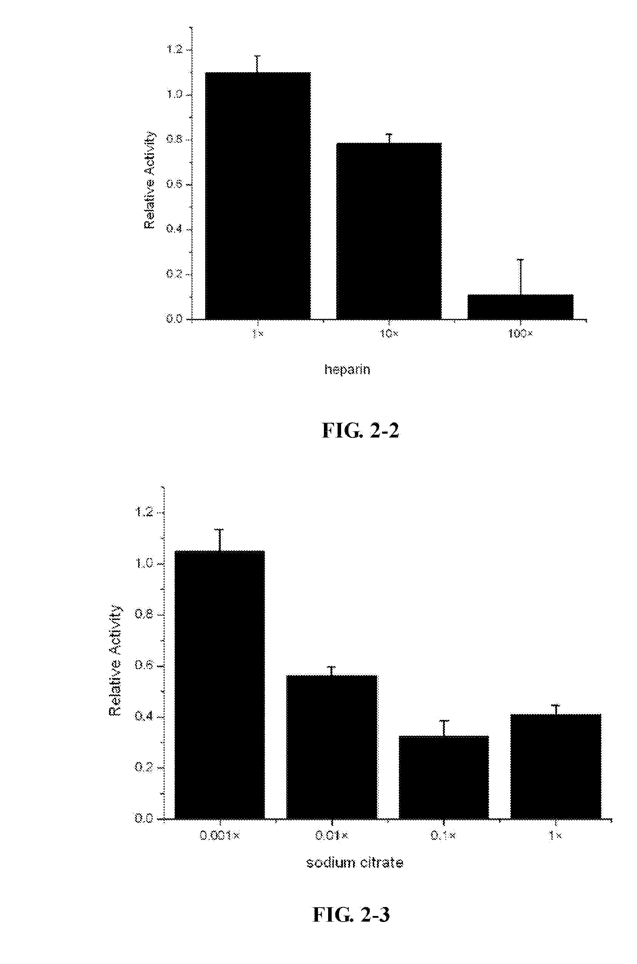

1.1 Impact of anticoagulant agent on the enzyme activity (fig. 2)
From the fig.2, with the increase of concentration of anticoagulant agent:

•EDTA did not seem to affect the ARS activity
•sodium heparin and sodium citrate have dramatic effect on the ARS activity where it is reduced with the increase of dosages

To ensure activities of the enzyme, we choose serum samples without anticoagulant agent treatment as experiment samples.

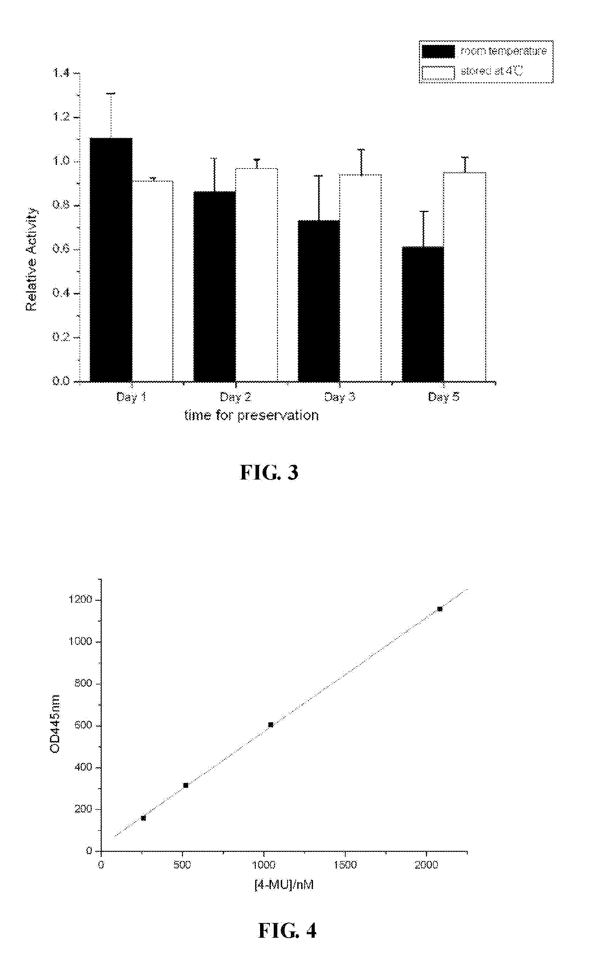

1.2 Impact of preservatives on the enzyme activity (fig. 3)
From fig. 3:

•under preservative condition at room temperature, the activity of the serum ARS enzyme is dramatically reduced every day, with about 10% reduction each day compared to the previous day
•at 4°C, the activity of the serum ARS enzyme is insignificantly changed with time
•by freezing-thawing for 10 times, the activity is reduced by about 20%
For not affecting the activity of enzyme, we choose the temperature of -20°C.

1.3 Reaction parameters determined by basic enzyme experiments:

•OD-[P] is linear
•[P]-time is linear in early phase
•initial reaction speed v-[E] is linear (if [S]>>[E]0)

1.3.1 Determination of OD-[4-MU] standard curve: in order to ensure the fluorescence reading is linear with 4-MU concentrations, fig. 4 was drawn (We obtained consistent results in repeated experiments) :

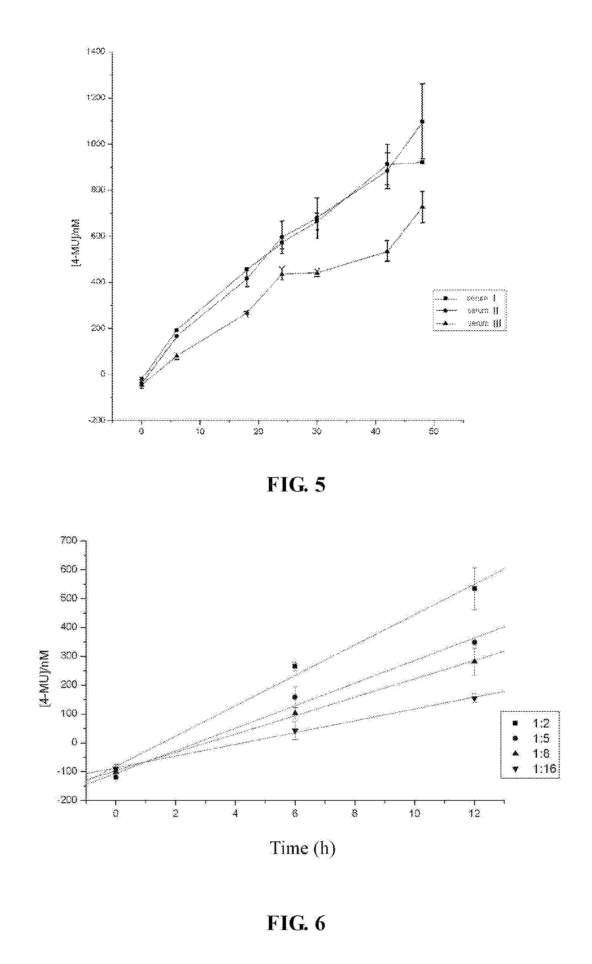

1.3.2 Determination of [4-MU]-time curve:
We selected 3 serum samples to determine the optimal reaction time, the result shows (fig. 5): the reading of [4-MU] is linear when the enzyme reaction time is within 36h;
In order to ensure values of [4-MU] readings from experimental measurements are within linear range and are obvious different from background (comparative) value, the reaction time is set as 24h. Therefore, all our experiments using serum samples described below used 24 hours as the reaction time if not specified elsewhere.

1.3.3 [4-MU]-time curve of serum samples at different diluting conditions (fig. 6)
In order to ensure the dilution ratio of serum samples is within linear range, the ratio of 1:8 is selected, then 20$\mu$L of which is added to the reaction system.

2 Determination of reaction parameters: the serum is diluted to 1/8, take 20$\mu$l for enzymatic reaction, fluorescence reading is measured after 24h, and at the same time, OD-[4-MU] standard curve is determined, finally ($OD_{sample}$-$OD_{control}$(water)) is converted into [4-MU]

(2) Experimental steps:

1 Serum sample examination

Take 5mL blood into a vacuum cuvette with separation gel and without anticoagulant agent, standing for about 30min at temperature, then centrifugate it with horizontal centrifugal machine for 10min (3000r/min). The supernatant serum is sucked/poured out to a 1.5mL EP tube/or other type of unused tubule, cool it at -20°C before use.
In activity determination, take 50$\mu$l serum into 350$\mu$l 100mM sodium acetate buffer (pH 5.4).
20$\mu$l described diluted serum is added to 180$\mu$l reaction system (containing 100mM sodium acetate, pH 5.4, 8mM 4-MUS).
It is placed in a thermal container (37°C) for 24h, then the reaction is stopped by adding 1 ml stop solution (500mM sodium carbonate/sodium bicarbonate buffer, pH 10.7), finally fluorescence of each sample is determination (365nm excitation, recording 445-450nm emitting light value).
Standard sample 4-MU (pure product) is used for OD-[4-MU] standard curve, and serum reaction readings are converted according to this standard curve to yield [4-MU] for each reaction.
Based on the described [4-MU], the activity of the serum ARS or its isozymes is determined as the amount of 4-MU produced by 1 L serum protein in 1 minute.

2 Urine sample examination

Take about 3mL morning urine sample into sterilized EP tube. In case of with impurity, it is centrifuged for 10min (3000r/min) to remove impurity and stored at - 20°C.
In activity determination, take 40$\mu$L urine sample with micropipettor into reaction tube, add 40$\mu$L precipitator (30% lead acetate solution), mix well, let the samples stand at 4°C for 5min. It is then centrifuged for 5min at room temperature (3000r/min).
The supernatant is taken away with micropipettor. Add 40$\mu$L substrate preparation (containing 100mM sodium acetate, pH 5.4, 8mM 4-MUS) to the tube, mix well, leave no coagulum, cultivate for at 37°C 30-40min, the reaction time is recorded as T. Thereafter, the reaction tube is quickly placed on ice, added by 200$\mu$L stop solution (500mM sodium carbonate/sodium bicarbonate buffer, pH 10.7), mix well. The reaction tube is centrifugated for 1 min at room temperature (6000-8000r/min).

Take 200μL post-reaction mixed urine sample and zero control solution (100mM sodium acetate buffer, pH 5.4) for fluorescence determination (365nm excitation, recording 445-450nm emitting light value).

Standard sample 4-MU (pure product) is used for OD-[4-MU] standard curve, and urine reaction readings are converted according to this standard curve to yield [4-MU] for each reaction.

According to the above [4-MU], the activity of the urine ARS or its isozymes is determined as the amount of 4-MU produced by 1 L urine protein in 1 minute.

(3) Data processing I:

Measure the standard sample in buffers as described above (containing sodium acetate and stop solution component in same concentration), i.e. OD of 4-MU (0.003mM), recorded as ODs

Determine the OD of control reaction (reaction of 20μl sodium acetate buffer to replace serum/reaction of 40μl sodium acetate buffer to replace urine), which is recorded as ODO

$$(ODn-OD0)/[4-MU]n=ODs/[4-MU]s$$

$$[4-MU]n=(ODn-OD0)*0.003mM/ODs$$

It is possible to calculate the amount of product 4-MU produced from reaction of sample n, i.e. amount of substrate 4-MUS joining the reaction.

It is possible to obtain the activity of sample n, i.e. molar number of substrate that can be converted by 1 L enzymatic protein in 1min.

The equation is [4-MU]n/(L*24*60)

The unit is converted into nmol/min/L

Data processing II:

Measure the standard sample in buffers as described above (containing sodium acetate and stop solution component in same concentration), i.e. OD of 4-MU (0.003mM), recorded as ODs

Determine the OD of control reaction (reaction of 20μl sodium acetate buffer to replace serum/reaction of 40μl sodium acetate buffer to replace urine), which is recorded as ODO

$$(ODn-OD0)/[4-MU]n=ODs/[4-MU]s$$

$$[4-MU]n=(ODn-OD0)*0.003mM/ODs$$

It is possible to calculate the amount of product 4-MU produced from reaction of sample n, i.e. amount of substrate 4-MUS joining the reaction.

Determine the protein amount of sample n: detected with bovine serum albumin (BSA) as control, ultraviolet spectrophotometer at $\lambda$ = 280nm to obtain the protein concentration of sample n that is recorded as Mn, from which the protein amount of sample n can be calculated as Mgn.

It is possible to obtain the specific activity of sample n, i.e. molar number of substrate that can be converted by 1 mg enzymatic protein in 1min.

As formula: [4-MU]n/(Mgn*24*60)

The unit is converted into nmol/min/mg

Data processing III:

The fluorescence values of 4-MU standard samples B1, B2, B3 and B4 are used as ordinate(y), the corresponding concentration values of standard solution (in nM) as abscissa (x), draw the standard curve through the origin: y=kx

Calculate net fluorescence value of the samples Ai'= Ai-A0, which is substituted into the above equation as y to obtain concentration of 4-MU in post-reaction solution, i.e. [4-MU]i, in nM

$$[4-MU]_i = \frac{A_i - A_0}{k}$$

Enzymatic activities of samples are calculated as below:

$$\frac{[4-MU]_i \times 10^{-3} \, \mu mol \cdot L^{-1} \times 240 \mu L}{T \min \times 40 \mu L}$$ (suitable for urine sample, in which 240µL is the total volume of post-reaction solution, 40uL is initial urine volume)

$$\frac{[4-MU]_i \times 10^{-3} \, \mu mol \cdot L^{-1} \times 240 \mu L}{T \min \times 0.4 \mu L}$$ (suitable for serum sample, in which 240µL is the total volume of post-reaction solution, 0.4µL is initial serum volume, i.e. add 4µL 1/10 diluting serum)

Value unit can be converted into U/L, in which $1U = 1\mu mol \cdot min^{-1}$

(4) Instruments for the experiments:

Horizontal centrifugal machine: Heraeus Labofuge 400R
Instrument for examination: it can detect fluorescence signal at 365nm of excitation wavelength and 445-450nm of detecting wavelength
Electro-thermostatical incubator: Tianjin Zhonghuan Experimental Circuit Ltd.
Vortex shaker: IKA MS2 Minishaker
Pipetman: Gilson 1mL, 200µl, 20µl

(5) Experimental data:

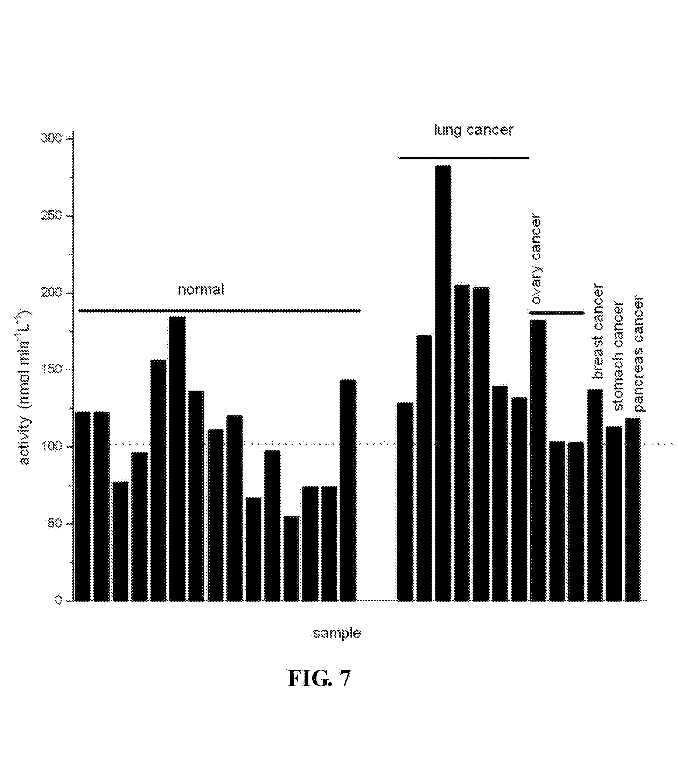

1. Measurement of the ARS activity in each serum sample from normal human (15 cases) and patients with cancer (7 cases of lung cancer, 1 case of stomach cancer, 3 cases of ovary cancer, 1 case of pancreas cancer, 1 case of breast cancer) is repeated for 3 times. Refer to fig. 7 for all the data.

Table 1

| Normal | | Cancer | |
|---|---|---|---|
| No. | Activity | Type | Activity |
| 1 | 122.9998 | | 128.5012 |
| 2 | 122.6639 | | 172.172 |
| 3 | 77.29412 | | 282.4619 |
| 4 | 96.07889 | Lung cancer | 204.933 |
| 5 | 156.0704 | | 203.5849 |
| 6 | 184.3498 | | 139.4111 |
| 7 | 136.4092 | | 131.9811 |
| 8 | 111.0366 | | 182.2981 |
| 9 | 120.3559 | Ovary cancer | 103.233 |
| 10 | 67.19366 | | 102.8881 |
| 11 | 97.60397 | Breast cancer | 137.2793 |

(continued)

| Normal | | Cancer | |
|---|---|---|---|
| No. | Activity | Type | Activity |
| 12 | 54.82057 | Stomach cancer | 112.9829 |
| 13 | 74.34256 | Pancreas cancer | 118.5946 |
| 14 | 74.15009 | | |
| 15 | 143.6181 | | |

2. Average of the ARS activity in serum samples from normal human and patients with cancer

Table 2

| | Normal human serum | Serum from patients with cancer |
|---|---|---|
| Number of sample | 15 | 13 |
| Average activity (nmol/min/L) | 109.2658±36.6046 | 155.4093±51.9724 |

T-test is completed for normal human serum (15 samples in total) and serum from patients with cancer (13 samples in total) to obtain p=0.005415<0.01.

3. Refer to fig. 2 for the sensitivity-specificity correlation corresponding to experimental data.

4. Determination of standard :

According to the correlation of sensitivity-specificity, we choose cutoff values with relative high sensitivity and specificity as standards.

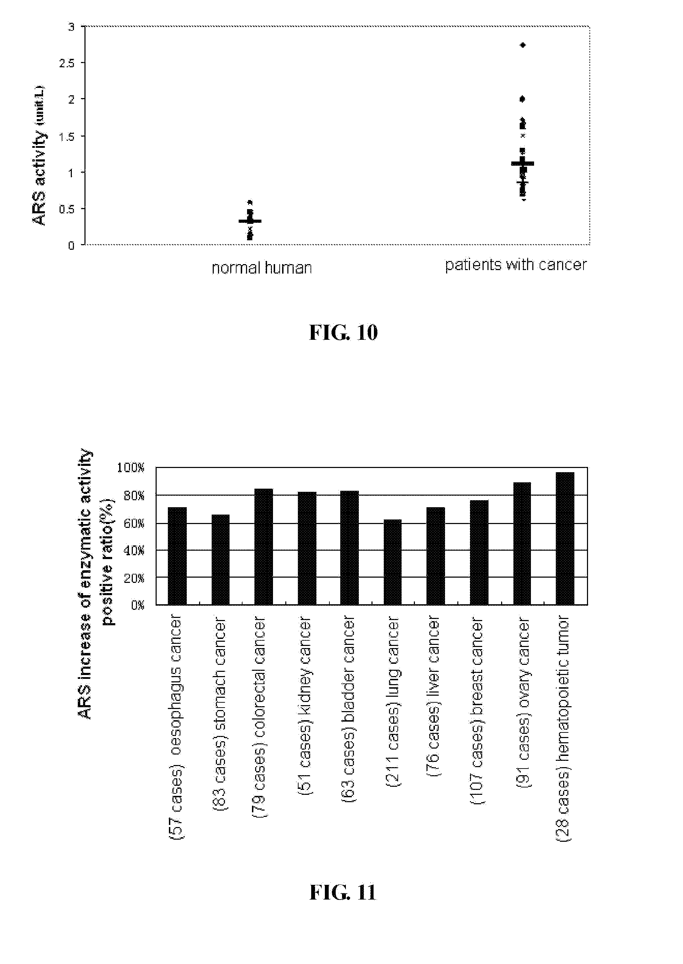

5. Measurement of the ARS activity in each urine sample from normal human (54 cases) and patients with cancer (12 cases of lung cancer, 10 cases of stomach cancer, 13 cases of colorectal cancer, 9 cases of oesophagus cancer, 5 cases of kidney cancer and 5 cases of breast cancer) is repeated for 3 times. Refer to fig. 10 for all the data.

As the figure shows, the activities of arylsulphatase in urine samples from patients with cancer are dramatically higher than that in normal human, indicating that urine ARS activity can be used as a marker for identifying tumor from normal samples.

6. The activities of arylsulphatase in urine samples from patients with cancer(57 cases of oesophagus cancer, 83 cases of stomach cancer, 79 cases of colorectal cancer, 51 cases of kidney cancer, 63 cases of bladder, 211 cases of lung cancer, 76 cases of liver cancer, 107 cases of breast cancer, 91 cases of ovary cancer, 28 cases of hematopoietic tumor) are examined, the sensitivities in different cancer types (true positive cases/(true positive cases + false positive cases)) are shown in fig. 11.

Among many urine samples from patients with different types of cancers examined, the average sensitivity of the arylsulphatase activities is not less than 80%, indicating that abnormal increases of the ARS activities in urine samples are common in patients with cancer.

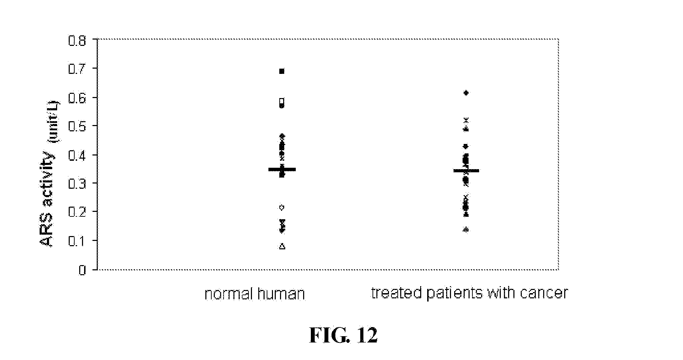

7. Measurement of the ARS activity in each urine sample from normal human (29 cases) and cancer patients after treatments (7 cases of lung cancer, 9 cases of oesophagus cancer, 9 cases of liver cancer, 2 cases of kidney cancer, 3 cases of ovary cancer, 1 case of hypopharynx cancer and 2 cases of hematopoietic tumor) is repeated for 3 times. Refer to fig. 12 for all the data.

We observed that in cancer patients after therapeutic treatments the urine ARS activity was dramatically reduced to the level close to that of normal controls (fig. 12). Therefore, the change in the ARS activity levels in urine samples can be used for therapeutic effect monitoring for cancer patients. If the treatment is effective, ARS activity value should be reduced than that before treatment till close to the level of normal people. The change in the ARS activity level in urine samples can also be used for monitoring cancer reoccurrence in patients after treatments.

## Claims

1. A kit for assaying activities of arylsulphatase or its isozymes, including 4-methyl umbelliferone sulfate and 4-methyl umbelliferone.

2. The kit according to claim 1, which is used for detecting cancer or other diseases and corresponding initial screening, therapeutic effect monitoring and recurrence inspecting.

3. The kit according to claim 1, in which the cancer is lung cancer, stomach cancer, ovary cancer, pancreas cancer, breast cancer, liver cancer, oesophagus cancer, intestines cancer, nasopharynx cancer, kidney cancer, bladder cancer, lymph cancer, cholecyst cancer, ampullae cancer, penis cancer, testicle cancer, throat cancer, hypothyroid cancer, prostate cancer, palace cancer, colorectal cancer, bile duct cancer, parenchyma sarcoma, spermatogonium tumor, Ewing's sarcoma, leukaemia, osteosarcoma, encephaloma or malignant lymphoma.

4. The kit according to claim 3, in which the cancer is lung cancer, stomach cancer, ovary cancer, pancreas cancer, breast cancer, liver cancer, oesophagus cancer, kidney cancer, bladder cancer, lymph cancer, colorectal cancer, leukaemia or malignant lymphoma.

5. The kit according to claim 1, in which the biological samples to be used are human whole blood, serum, urine, saliva, phlegm, lymph fluid or other tissue fluid.

6. The kit according to claim 5, in which the biological samples to be used are human serum.

7. The kit according to claim 5, in which the biological samples to be used are human urine.

8. The kit according to claim 1, in which the 4-methyl umbelliferone sulfate is within the range of 0.1-100mM.

9. The kit according to claim 8, in which the 4-methyl umbelliferone sulfate is within the range of 1-10mM.

10. The kit according to claim 8, in which the 4-methyl umbelliferone sulfate is 8mM.

11. The kit according to claim 1, in which the 4-methyl umbelliferone is within the range of 1-10000nM.

12. The kit according to claim 10, in which the 4-methyl umbelliferone is within the range of 100-5000nM.

13. The kit according to claim 10, in which the 4-methyl umbelliferone is within the range of 100-2000nM.

14. The kit according to claim 6, including a buffer and a stop solution.

15. The kit according to claim 7, including a buffer, a precipitator and a stop solution.

16. The kit according to claim 14 or 15, in which the buffer is sodium acetate.

17. The kit according to claim 16, in which the sodium acetate is within the range of 1-10000mM, and the pH value is within the range of 1-14.

18. The kit according to claim 17, in which the sodium acetate is within the range of 10-1000mM, and the pH value is within the range of 2-7.

19. The kit according to claim 17, in which the sodium acetate is 100mM, and the pH value is 5.4.

20. The kit according to claim 14 or 15, in which the stop solution is sodium carbonate/sodium bicarbonate or sodium carbonate/glycin solution.

21. The kit according to claim 20, in which the sodium carbonate/sodium bicarbonate or sodium carbonate/glycin solution is within the range of 1-50000mM, the pH value is within the range of 1-14.

22. The kit according to claim 21, in which the sodium carbonate/sodium bicarbonate or sodium carbonate/glycin solution is within the range of 10-5000mM, the pH value is within the range of 7-12.

23. The kit according to claim 21, in which the sodium carbonate/sodium bicarbonate or sodium carbonate/glycin solution is within the range of 500mM, the pH value is 10.7.

24. The kit according to claim 15, in which the precipitator is lead acetate.

25. The kit according to claim 24, in which the lead acetate is within the range of 1%-90% (w/v).

26. The kit according to claim 25, in which the lead acetate is within the range of 10%-40% (w/v).

27. The kit according to claim 25, in which the lead acetate is 30% (w/v).

4-methyl umbelliferone sulfate, 4-MUS                4-methyl umbelliferone, 4-MU

**FIG. 1**

**FIG. 2-1**

**FIG. 2-2**

**FIG. 2-3**

**FIG. 3**

**FIG. 4**

**FIG. 5**

Time (h)

**FIG. 6**

FIG. 7

FIG. 8

FIG. 9

**FIG. 10**

**FIG. 11**

FIG. 12

| INTERNATIONAL SEARCH REPORT | International application No. |
|---|---|
| | PCT/CN2008/000081 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: G01N33, C12Q1

Documentation searched other than minimum documentation to the extent that such documents are included in the fields searched

Electronic data base consulted during the international search (name of data base and, where practicable, search terms used)

CNPAT, WPI, EPODOC, PAJ: arylsulphatase, arylsulfatase, umbelliferone, umbelliferyl sulfuate

**C. DOCUMENTS CONSIDERED TO BE RELEVANT**

| Category* | Citation of document, with indication, where appropriate, of the relevant passages | Relevant to claim No. |
|---|---|---|
| X | JP2004305008A (SEIKAGAKU KOGYO CO LTD)  4 Nov. 2004(04.11.2004) , see paragraph [0059] | 1, 5-27 |
| Y | | 2-4 |
| X | Helene Christomanou et al., A sensitive fluorescence assay for the simultaneous and separate determination of arylsulphatases A and B, Clinica Chimic Acta,  1977, vol. 79. page 527-531. see the section of material and method | 1, 5-14, 16-22 |
| Y | | 2-4 |

☒ Further documents are listed in the continuation of Box C.   ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
|---|---|
| 9 Apr. 2008 (09.04.2008) | **24 Apr. 2008 (24.04.2008)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>WANG, Lihua<br>Telephone No. (86-10)62085676 |

Form PCT/ISA/210 (second sheet) (April 2007)

| INTERNATIONAL SEARCH REPORT | International application No. |
|---|---|
| | PCT/CN2008/000081 |

C (Continuation). DOCUMENTS CONSIDERED TO BE RELEVANT

| Category* | Citation of document, with indication, where appropriate, of the relevant passages | Relevant to claim No. |
|---|---|---|
| X | Rudi Mercelis et al., Arylsulphatases A and B in human diploid fibroblasts: differential assay with 4-methylumbelliferylsulphate and AgNO3, Clinica Chimic Acta, 1979, vol.93, page 85-92, see the section of material and method | 1, 5-14, 16-22 |
| Y | | 2-4 |
| Y | WANG, Mingxun et al., Observation of arylsulfatase activities in urines of patients suffering from blood disease, Journal of Bengbu Medical College, 1993, vol. 18, no. 1, page 56-57, see the section of discussion | 2-4 |
| Y | CHEN Tiehe et al., Clinical application of detecting activities of arylsulphatase B in morning urines, Journal of clinical laboratory science, 1996, vol. 14, no. 1, page 24-25, see the section of discussion | 2-4 |
| A | CHEN Tiehe et al., separation of arysulphatases in urines by a precipitation method and detection of activities thereof, Journal of clininal laboratory science, 1995, vol. 13, no. 4, page 174-175, see the section of material and method | 1-27 |

Form PCT/ISA/210 (continuation of second sheet ) (April 2007)

**INTERNATIONAL SEARCH REPORT**
Information on patent family members

| International application No. |
| --- |
| PCT/CN2008/000081 |

| Patent Documents referred in the Report | Publication Date | Patent Family | Publication Date |
| --- | --- | --- | --- |
| JP2004305008A | 2004-11-4 | None | |

Form PCT/ISA/210 (patent family annex) (April 2007)

**INTERNATIONAL SEARCH REPORT**

International application No.

PCT/CN2008/000081

According to International Patent Classification (IPC) or to both national classification and IPC

    C12Q1/44    (2006.01) i

    G01N33/573  (2006.01) i

Form PCT/ISA/210 (extra sheet) (April 2007)

EP 2 085 483 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Wozniak, A. et al.** *Neoplasma,* 2002, vol. 49 (1), 10-5
- **Gasa, S. et al.** *Cancer Res,* 1980, vol. 40 (10), 3804-9
- **Rozwodowska, M. et al.** *Pol Merkuriusz Lek,* 2004, vol. 17 (99), 252-4
- **Chetrite, G.S. et al.** *Anticancer Res,* 2005, vol. 25 (4), 2827-30
- **Dzialoszynski, L.M. et al.** *Clin. Chim. Acta,* 1966, vol. 14 (4), 450-3
- **Uchimura, K. et al.** *Methods In Enzymology,* 2006, vol. 416, 243
- **Sherman, W.R. et al.** *Biochem J.,* 1967, vol. 102, 905